(19) Europäisches Patentamt
European Patent Office
Office européen des brevets

(11) EP 1 785 431 B1

(12) **EUROPEAN PATENT SPECIFICATION**

(45) Date of publication and mention of the grant of the patent:
**20.01.2016 Bulletin 2016/03**

(21) Application number: **05781472.5**

(22) Date of filing: **30.08.2005**

(51) Int Cl.:
***C07K 1/22*** *(2006.01)* ***C08J 7/12*** *(2006.01)*
***A61L 31/00*** *(2006.01)* ***A61F 2/06*** *(2006.01)*
***A61J 3/00*** *(2006.01)* ***A61M 1/18*** *(2006.01)*
***A61M 25/00*** *(2006.01)* ***C08L 101/00*** *(2006.01)*

(86) International application number:
**PCT/JP2005/015704**

(87) International publication number:
**WO 2006/025352 (09.03.2006 Gazette 2006/10)**

(54) **FRACTIONATION APPARATUS**

FRAKTIONIERUNGSVORRICHTUNG

APPAREIL DE FRACTIONNEMENT

(84) Designated Contracting States:
**AT BE BG CH CY CZ DE DK EE ES FI FR GB GR HU IE IS IT LI LT LU LV MC NL PL PT RO SE SI SK TR**

(30) Priority: **30.08.2004 JP 2004250417**
**04.03.2005 JP 2005060269**

(43) Date of publication of application:
**16.05.2007 Bulletin 2007/20**

(73) Proprietor: **TORAY INDUSTRIES, INC.**
**Tokyo 103-8666 (JP)**

(72) Inventors:
- **TANAHASHI, Kazuhiro**
**Otsu-shi, Shiga 520-0801 (JP)**
- **TAKAHASHI, Hiroshi**
**Kyoto-shi, Kyoto 607-8088 (JP)**
- **SUGAYA, Hiroyuki**
**Otsu-shi, Shiga 520-2101 (JP)**
- **WADA, Shigehisa**
**Otsu-shi, Shiga 520-0842 (JP)**

(74) Representative: **Webster, Jeremy Mark et al**
**Mewburn Ellis LLP**
**City Tower**
**40 Basinghall Street**
**London EC2V 5DE (GB)**

(56) References cited:
**WO-A1-00/27496** **WO-A2-2004/026931**
**JP-A- 1 026 656** **JP-A- 2 203 933**
**JP-A- 2 222 424** **JP-A- 5 262 664**
**JP-A- 10 059 863** **JP-A- 2000 351 814**
**US-A1- 2003 068 317**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 1 785 431 B1

## Description

TECHNICAL FIELD

The present invention relates to a fractionation device which prepares an analyzing solution by separating biological components such as proteins and/or peptides from a solution containing proteins and/or peptides, in particular, from a body fluid such as blood and urine.

BACKGROUND ART

Recently, proteome analysis research (proteomics) has begun to draw attention as postgenome research. Since it is a very likely supposition that proteins, gene products, are more directly linked with symptoms of diseases than genes, it has been highly expected that research findings and achievements of proteome analysis of thoroughly investigating proteins can widely be applicable for diagnosis and medical care. Moreover, it is highly possible to find many proteins causing diseases and factors relevant to diseases, which cannot be found by genome analysis.

High speed structural analysis has been made possible by MS (mass spectrometer) and technically it has greatly contributed to rapid advancement of proteome analysis, and practical application of MALDI-TOF-MS (matrix assisted laser desorption ionization time-of-flight mass spectrometry) has enabled ultramicroanalysis of polypeptides to be performed at a high throughput, and that makes it possible to identify even trace proteins which have not been detected conventionally and accordingly becomes a powerful tool for searching factors relevant to diseases.

The primary purpose of clinical application of the proteome analysis is to find biomarker proteins induced or eliminated by diseases. The biomarker behaves in relation to symptoms of diseases, so that it can be a marker for diagnosis and also highly possibly becomes a target for producing pharmaceuticals. That is, since the findings and achievements of proteome analysis are highly possibly applicable to find a diagnosis marker and a target for producing pharmaceuticals rather than specified genes, it can be said that proteome analysis becomes a key technology (an evidence) for diagnosis and medical care in the postgenome era and since the identifiedbiomarker directly brings profits to patients, that is, evaluation of response to the pharmaceuticals and speculation of side effect expression, it can be said that this technique plays an important role in promoting so-called tailor-made medical care (order-made medical care).

In the case proteome analysis (clinical proteomics) is to be introduced in clinical researches, it is required to quickly and reliably analyze a large number of samples and moreover, since each clinical sample is slight in the amount and very precious, it is required to quickly carry out the high resolution, high sensitivity, and highly functional measurement. Mass spectrometry has considerably propelled the analysis and the characteristics of mass spectrometers, that is, ultrahigh sensitivity and high throughput, have greatly contributed to the analysis. However, although the techniques and appliances have been improved swiftly, the present situation is not yet ready to simply and quickly carry out proteome analysis in a clinical field.

It is assumed that there are 100,000 or more kinds of human proteins and as many as about 10,000 kinds of proteins are contained in serum and the concentration of the total proteins in the serum is about 60 to 80 mg/mL. The proteins contained in the serum in a large amount are albumin (molecular weight: 66 kDa), immunoglobulin (150 to 1000 kDa), transferrin (80 kDa), haptoglobin (>85 kDa), and lipoprotein (several 100 kDa) and all of them exist respectively in a large amount (>mg/mL). On the other hand, many of physiologically active proteins such as peptide hormones, interleukins, and cytokines regarded to be biomarkers of symptoms and factors relevant to diseases exist in a trace amount (< ng/mL). The contents are no more than nano to pico level as compared with those of the high content components with high molecular weights. In terms of the size of proteins, 70% or more in all kinds of proteins have a molecular weight of 60 kDa or lower and the above-mentioned biomarker proteins existing in a trace amount are almost all included in this range (for example, Non-Patent Document No. 1). Since these proteins are partially excreted to urine through a kidney, not only blood but also urine may be measured as a sample.

To carry out proteome analysis by general serologic investigation, it is at first essential to remove high molecular weight components with a molecular weight of 60,000 or higher, which become obstacles to detection of trace components relevant to disease. It is also essential to recover separated trace components relevant to the diseases and having a molecular weight less than 60,000 as many as possible.

Presently, high performance liquid chromatography (LC) and 2-dimensional electrophoresis (2 dimensional polyacrylamide gel electrophoresis: 2D-PAGE) have been employed as means for separation and removal of the high molecular weight proteins. Here, with respect to already practically utilized products or disclosed techniques for albumin as a main object substance, there is a carrier in which an affinity ligand such as a blue dye is immobilized (for example, "Montage Albumin Deplete Kit" made by Japan Millipore Co., Ltd., "AffiGel Blue" gel, made by Bio-Rad Microscience Ltd.), a centrifugal tubular apparatus used for fractionating the high molecular weight components by centrifugal filtration (for example, "Amicon Ultra", made by Japan Millipore Co., Ltd.), a method of fractionation by electrophoresis principle disclosed in Japanese Patent Application National Publication (Laid-Open) No. 2002-542163 (Patent Document No. 1)

(for example, "Gradiflow" system, made by Gradipore Co., Ltd.), a traditional precipitation method such as ethanol precipitation by Cohn, and a method of fractionation by chromatography (for example, Non-Patent Document No. 2) . In general, these techniques have been conducted as pretreatment operations of mass spectrometry.

Upon dealing with proteins, a problem of non-specific adsorption of proteins onto a substrate surface is always raised. The non-specific adsorption onto the substrate surface causes not only deviations in the analysis results due to a reduction in proteins, but also a serious problem of a loss of analysis target proteins; therefore, it is necessary to prevent the non-specific adsorption. In general, the rate of reduction in proteins due to the non-specific adsorption of proteins depends on the total concentration of proteins in a solution, and the lower the total concentration of proteins, the greater the rate of reduction in proteins becomes. In particular, in the case when a trace component relevant to diseases is analyzed through mass spectrometry in the above-mentioned proteome analysis, since no device is available to which a non-specific adsorption suppressing treatment has been applied, among existing pretreatment devices, the total concentration of proteins in a fractionated solution obtained by excluding components that interfere with the detection is extremely low, resulting in problems of the reduction and loss due to non-specific adsorption of trace biomarker proteins.

Mainly two countermeasures are proposed so as to solve this problem of the loss due to adhesion of proteins and peptides. One is a method in which a compound for suppressing adsorption is added to a solution containing biological components and the other is a method in which a biological component non-adsorption treatment is carried out on the substrate surface. A method in which a blocking agent is added is proposed as a representative method for the former method. The blocking agent forms a solution of albumin or casein, and provides a method by which adsorption of necessary biological components is suppressed through competitive adsorption. To provide the competitive adsorption, the concentration of the blocking agent is generally kept higher than the concentration of the necessary biological components. Therefore, when this is used for analysis purposes, there are the risks that the blocking agent might intervene with the analysis and that it might cause a structural change in the biological component even in the case of a slight amount of addition. In addition to the blocking method, another method is proposed in which a surface active agent or an organic solvent is added; however, this method also causes problems of a failure in the analytic system and denaturing following the structural change in the biological component, in the same manner as the blocking agent.

A hydrophilizing treatment on a substrate surface is generally used as the non-adsorption treatment of the substrate surface. The hydrophilizing treatment includes several methods. For example, Patent Document 2 has disclosed a method in which a hydrophilic compound, such as a 2-methacryloyloxyethylphosphorylcoline copolymer (hereinafter, referred to simply as MPC), is introduced onto a substrate through coating treatment. Patent Documents 3 and 4 have disclosed methods in which a hydrophilic compound is introduced thereto through a graft treatment. There are also methods in which a hydrophilic functional group is directly formed on a substrate surface by using processes, such as a reactive ion etching process, a plasma process and an ion cluster beam process.

However, with respect to the substrate that has been processed through a conventional substrate surface treatment method, although an adsorption suppressing effect for biological components is confirmed when made in contact with a solution containing proteins and peptides with a high concentration, a reduction and a loss of biological components due to adsorption still occur in the case when it is made in contact with a solution containing biological components with a low concentration, failing to provide a method in such a level as to sufficiently solve the problems.

With respect to the technique using a coating treatment by the use of a hydrophilic polymer, in the case when a solvent that has been used for the hydrophilic polymer solution is further made in contact with the treated substrate, the hydrophilicity might be lowered due to separation of the coating or the like. Moreover, with respect to the treatment devices for analysis, separation and the like, eluted hydrophilic polymer might cause obstacles to the succeeding analysis.

The hydrophilizing process by the hydrophilic polymer grafting makes it possible to improve the hydrophilicity in proportion to the amount of graft; however, since, upon high concentration of the hydrophilic polymer solution to be treated, the hydrophilic polymers are mutually crosslinked with one another three dimensionally, the motility of the hydrophilic polymer is lowered to cause a reduction in the adhesion suppressing effects for the biological components.

Moreover, the reactive ion etching process, the plasma treatment and the ion cluster beam treatment make it possible to easily conduct a hydrophilizing process onto the outer surface of a substrate and one surface of a plate-shaped substrate; however, since it is difficult for these treatments to conduct a hydrophilizing process on a portion that forms a shadowed portion from plasma, iron cluster beams or the like, these treatments are not suitable for hydrophilizing multiplesurfaces, such as both of the surfaces of a plate-shaped substrate and inner and outer surfaces of a hollow shaped substrate at one time. Furthermore, since the adsorption property of biological components onto a substrate depends on the surface state of a contact portion to the biological components, in general, as the hydrophilicity of the surface becomes higher, and as the motility of hydrophilic molecules fixed onto the surface becomes higher, the adsorption of the biological components onto the substrate surface is further suppressed. It is assumed that the hydrophilic molecules having high motility exclude biological components such as proteins and platelets by the molecular movements thereof. The hydrophilizing process by the reactive ion etching process, the plasma treatment or the ion cluster beam treatment depends on generation of a hydrophilic functional group such as a hydroxyl group on the substrate surface; therefore, since the motility of hydrophilic molecules is low in comparison with the hydrophilizing process by the introduction of a

hydrophilic polymer onto the substrate surface, the adhesion suppressing effects for the biological components is low, resulting in a failure to provide a desirable method. Moreover, since these processes sometimes cause a high temperature during the treatment, the substrate tends to be denatured, also resulting in a failure to provide a desirable method.

In this manner, since the technique for the adsorption suppressing treatment has not yet been achieved, there is no device to which a proper adsorption suppressing treatment is applied among fractionation devices used for proteins and/or peptides.

[Patent Document No. 1] No. 2002-542163; [Patent Document No. 2] JP-A) No. 2003-130882; [Patent Document No. 3] JP-A No. 58-40323; [Patent Document No. 4] JP-B. 3297707

[Non-Patent Document No. 1] Anderson NL, Anderson NG, "The human plasmaproteome: history, character, and diagnostic prospects", Molecular & Cellular Proteomics, USA, The American Society for Biochemistry and Molecular Biology, Inc., (2002) vol. 1, p845-867:

[Non-Patent Document No. 2] The Japanese Biochemical Society, "New Biochemical Experiments (vol. 1)", Proteins (1) separation-refining-characteristics", TOKYO KAGAKUDOZIN CO., LTD. (1990)

In addition WO2004/026931 and US 2003/0068317 disclose compositions comprising a base material having engrafted polymer brushes. WO 00/27496 concerns a chromatography method and column material.

PROBLEMS TO BE SOLVED BY THE INVENTION

As described above, upon conducting a clinical proteome analysis, it is necessary to carry out a pretreatment operation to eliminate high molecular weight proteins that interfere with mass analysis. There are only trace amounts of proteins that form targets of the proteome analysis, and since the total protein concentration is lowered by the pretreatment, the analysis target proteins are lost due to non-specific adsorption onto the substrate, resulting in a failure to carry out the proteome analysis stably. In order to carry out a stable proteome analysis, it is necessary to suppress the non-specific protein adsorption in the above-mentioned pretreatment, and also to provide a device that reduces the non-specific adsorption of proteins to the substrate, and can easily prepare an advantageous solution for analysis from which high molecular weight proteins have been eliminated.

In order to solve the above-mentioned problems, the present invention is provided with the following means:

1) A fractionation device used for proteins and/or peptides, comprising:

a substrate having a surface with which proteins and/or peptides are made in contact,

wherein the substrate has at least one portion of the surface where a hydrophilic polymer is grafted thereon by contacting the surface with an aqueous hydrophilic polymer solution having a hydroxyl group and by irradiating the surface with radioactive rays, and wherein at least one portion of the hydrophilic polymer is any one of polyvinyl alcohol, copolymers thereof and graft polymers thereof, such that the surface adsorbs bovine serum albumin in an amount of 50 $ng/cm^2$ or less with respect to the substrate surface, when a bovine serum albumin solution of 1000 $\mu$g/ml is made in contact therewith.

2) A fractionation device used for proteins and/or peptides, comprising:

a substrate having a surface with which proteins and/or peptides are made in contact,

wherein the substrate has at least one portion of the substrate surface where a hydrophilic polymer is grafted thereon by contacting the surface with an aqueous hydrophilic polymer solution having a hydroxyl group and by irradiating the surface with radioactive rays,

and wherein at least one portion of the hydrophilic polymer is any one of polyvinyl alcohol, copolymers thereof and graft polymers thereof, such that the surface adsorbs human $\beta$2-microglobulin in an amount of 3 $ng/cm^2$ or less when a protein aqueous solution consisting of human $\beta$2-microglobulin having a concentration of 200 ng/ml and bovine serum albumin having a concentration of 10 $\mu$g/ml is made in contact with the substrate surface.

3) A fractionation device which is used for proteins and/or peptides, comprising:

a means for supplying a solution containing proteins and/or peptides;

a means for separating proteins and/or peptides from the solution; and
a means for concentrating proteins and/or peptides in the solution,
wherein at least one portion of the surface of the fractionation device with which proteins and/or peptides are made in contact is grafted with hydrophilic polymer by contacting the surface with an aqueous hydrophilic polymer solution having a hydroxyl group and by irradiating the surface with radioactive rays and wherein at least one portion of the hydrophilic polymer is any one of polyvinyl alcohol, copolymers thereof and graft polymers thereof.

With the above-mentioned arrangement, it becomes possible to recover proteins that are targets for analysis without vain loss through a fractionating operation.

BRIEF DESCRIPTIONS OF THE DRAWING

Fig. 1 is a schematic view that shows a fractionation device used in example A1 in accordance with the present invention.

Moreover, reference numerals in the drawing are explained below:

| | |
|---|---|
| 100 | Injection pump; |
| 101 | Three-way valve; |
| 102 | First stage solution circulation channel (tube channel); |
| 103 | First stage pump |
| 104 | Treated solution recovery port of first stage membrane separation unit |
| 105 | First separation membrane module |
| 201 | Three-way valve |
| 202 | Second stage solution circulation channel (tube channel) |
| 203 | Second stage pump |
| 204 | Treated solution recovery port of second stage membrane separation unit |
| 205 | Second separation membrane module |
| 301 | Three-way valve |
| 302 | Third stage solution circulation channel (tube channel) |
| 303 | Third stage pump |
| 304 | Treated solution recovery port of third stage membrane separation unit |
| 305 | Third separation membrane module |
| 401 | Three-way valve |
| 402 | Fourth stage solution circulation channel (tube channel) |
| 403 | Fourth stage pump |
| 404 | Treated solution recovery port of concentration membrane unit |
| 405 | Concentration membrane module |
| 406 | Recovery three-way valve |
| 407 | Recovery container |

BEST MODE FOR CARRYING OUT THE INVENTION

A fractionation device for proteins and/or peptides in accordance with the present invention is a device used for separating these materials based upon the molecular weight or properties possessed by proteins or peptides. With respect to the method thereof, the molecular weight, ion interactive function, hydrophobic interactive function and biological interactive functions typically represented by antibodies can be utilized. In general, this device is provided with a supply means used for supplying a protein or peptide sample and a separation means used for carrying out the separation process. When a body fluid typically represented by serum, plasma and urine, or a solution containing proteins or peptides artificially prepared, or a cultured supernatant produced by the cell culture, as its own concentration, or as diluted with pH buffer solution etc., is put into the supply means, the specimen is conveyed by a transfer liquid, and separated by the separation means that is provided with a membrane, gel and adsorption material, and installed in the channel of the transfer liquid. The solution containing the target proteins or peptides, separated by the fractionation device of the present invention, is quantified by using ultraviolet absorption, fluorescent light, colorimetry, or the like, or concentrated by removing a moisture component contained together with proteins or peptides, if necessary, and then separately entrapped or recovered on a fixed amount basis. The means used for the quantifying, concentrating, separately entrapping and recovering processes are not necessarily required to be integrated into the means for the separation process; however, a device in which at least one means among the quantifying, concentrating, separately entrapping

and recovering means is coupled to the separation means is preferably used. In the case when a protein or peptide solution having a high concentration needs to be prepared by the request of the analyzer that is used after separating and further recovering the proteins or peptides, a device in which a concentrating means is continuously coupled to the separation means is preferably used. The means for conveying the proteins or peptides by using a transfer liquid is not particularly limited, and for example, passages, constituted by tubes, pipes or grooves and connected to a tube pump, a gear pump, a diaphragm pump, a syringe pump or the like, can be used.

In particular, in the case when the device is used for obtaining a sample used for pre-treatment for a proteome analysis, a solution that contains much micro-components required for the analysis is prepared from an original solution derived from a living body. The function is to remove proteins having high molecular weights (for example, molecular weights of 60,000 or more) and also to recover proteins having low molecular weights (for example, molecular weights of less than 15,000) that can form the target for the proteome analysis. Here, the device used for this purpose is preferably provided with a means selected from the group consisting of a means for separating protein, a means for concentrating the protein and a means for recovering proteins obtained through fractionation, and also provided with a means for conveying a protein solution.

The proteins having molecular weights of 60,000 or more correspond to albumin, immunoglobulin and transferrin in the case of blood. In the case of blood, the proteins of this type exist at a high content in all the protein components. With respect to the evaluation on functions of the fractionation device of the present invention, bovine serum albumin having molecular weights close to 60,000 was used as index. Moreover, the proteins having molecular weights of less than 15,000, which are proteins having comparatively small molecular weights among all kinds of proteins, have a comparatively low rate of existence with respect to the total proteins in the case of blood; however, there are many kinds of proteins of this type.

In the case when the fractionation device of the present invention has a separation means, the solution containing proteins or peptides is sometimes diluted upon carrying out a separating process. For example, when a specimen is separated through a membrane, the solvent is normally reduced. When the proteins or peptides come to have a higher concentration, deposits are laminated on the film surface to cause clogging in membrane holes, resulting in a reduction in the separating efficiency. In order to prevent this, a dilution solution is sometimes added thereto so as to prevent the protein concentration from increasing.

However, in the case when the target protein has a low concentration even after proteins that interfere with the analysis have been removed, the concentration might be lower than the detection limit of the analyzer. Therefore, when the concentration of a solution containing the target component after the separation is low, a concentrating means is preferably installed after the separation means so as to carry out a concentrating operation. Here, when only the solvent is removed, the salt concentration in the solution tends to increase too much with the result that the proteins might be denatured; therefore, it is preferable to remove salts simultaneously with the solvent. The main concentrating methods include: a method in which the solvent is evaporated or freeze-dried by heating or pressure-reducing; a method in which the solvent is removed through filtration or dialysis by using a separation membrane that preferentially permeates the solvent molecules; a method in which the solvent is absorbed and removed by using a water-absorbing gel or the like; a method in which a concentrating process is carried out through chromatography utilizing a molecule sieving effect, an ion interaction, a hydrophobic interaction, a hydrogen bond and a peculiar bond based upon affinity; and electrophoresis as well as centrifugal separation, and these methods may be combined with one another. The fractionation device of the present invention is preferably provided with a means capable of carrying out any of these operations.

With respect to the substrate of the present invention with which proteins or peptides are made in contact, examples thereof include: constituent elements to which the solution containing proteins or peptides are allowed to adhere in the means for supplying a sample containing the proteins or peptides, as well as in any of the means for carrying out any one of the operations including separation, adsorption, quantifying, concentrating, separately entrapping and recovering operations. In the present device, at least one portion of the substrate surface with which the specimen is made in contact is subjected to a hydrophilizing treatment, and most preferably, possibly all the substrate surface is subjected to a hydrophilizing treatment.

With respect to the material used for the substrate, polymer materials, which are chemically processed and surface-modified easily, and can be mass-produced at low costs through extrusion molding or injection molding, are preferably used. Examples of the polymer materials include: polypropylene, polyethylene, polystyrene, polycarbonate, polymethylmethacrylate, polysulfone, polyethersulfone, polyurethane, polyvinyl chloride, polyvinylidene chloride, polyacrylonitrile, cellulose, cellulose acetate, cellulose triacetate, polyamide, polyimide, polytetrafluoroethylene, vinyl alcohol-ethylene copolymer, silicon rubber, epoxy resin and phenolic resin.

In the fractionation device of the present invention, a surface on which proteins or peptides are hardly adsorbed is formed at least as one portion of the substrate surface with which the proteins or peptides are made in contact so that it becomes possible not only to restrain the adsorption loss, but also to further prevent a reduction in the separation performance due to adsorption, deposition and clogging of proteins or peptides, as further functions thereof. As a result, a high recovery rate has been achieved. In order to achieve these, the surface characteristics are set so that when a

solution containing 1000 μg/ml of bovine serum albumin is made in contact with an area in a range from 0.01 $cm^2$ or more to 10 $cm^2$ or less, the amount of adsorption of the bovine serum albumin onto the substrate surface is set to 50 $ng/cm^2$ or less. Moreover, when a protein aqueous solution consisting of human β2-microglobulin having a concentration of 200 ng/ml and bovine serum albumin having a concentration of 10 μg/ml is made in contact with the substrate surface, the amount of adsorption of the human β2-microglobulin onto the substrate is set to 3 $ng/cm^2$ or less. These surface characteristics are evaluated in the following method.

With respect to the adsorption evaluation of protein onto a substrate surface, the following description will discuss an operation in which a solution of bovine serum albumin (hereinafter, referred to simply as BSA) that is a generally-used protein, and is comparatively inexpensive, is used. With respect to the BSA solution, in order to restrain deviations in adsorption due to impurities and the like, a solution, prepared by dissolving BSA (ALBUMIN, BOVINE (A-7906), made by SIGMA) in a phosphoric acid buffering physiological saline solution (prepared by dissolving 9.6 g of PBS (-) Powder (162-21112) manufactured by NISSUI PHARMACEUTICAL CO., LTD in injection-use water (2069) available from OTSUKA PHARMACEUTICAL CO., LTD. so as to form a 1 liter solution, hereinafter, referred to simply as PBS) to be set to 1000 μg/ml, is used. Moreover, the area with which the BSA solution is made in contact with respect to the substrate is preferably set in a range from 0.01 $cm^2$ or more to 10 $cm^2$ or less because if it is too small, an accurate analysis might not be carried out, and because, in contrast, if it is too large, the amount of protein to be used becomes too large to cause degradation in the efficiency. More preferably, the range is set from 0.1 $cm^2$ or more to 5 $cm^2$ or less.

With respect to the face with which the BSA solution is made in contact, a face that is actually made in contact with a protein solution in the pre-treatment of the protein is used, and, for example, in the case when fragments etc. of the substrate are included, the adsorption of BSA onto the cross section or the like is not taken into consideration. With respect to the surface area, in the case when the substrate is neither a separation membrane nor a concentrationmembrane, if the center-line average roughness on the substrate surface is less than 0.1 μm, the increase in the area due to irregularities on the surface is not taken into consideration; however, if the center-line average roughness on the substrate surface is 0.1 μm or more, the increase in the area due to irregularities on the surface is taken into consideration. The measurements of the center-line average roughness are carried out by a method described in attached document 2 of JIS-B0601. The surface area in which the surface irregularities have been taken into consideration can be measured by a gas adsorption method such as a B.E.T method or a three-dimensional shape measuring microscope, such as an ultra-depth microscope "VK 9500" made by KEYENCE CORPRATION.

In contrast, in the case when the substrate is a hollow fiber separation membrane or a hollow fiber concentration membrane, the membrane area is calculated in the following manner: a filtration process is carried out by using a module filled with 1.5 $m^2$ of a membrane that is a subject under conditions of use of a solution flow rate of 200 ml/min with a filtration flow rate of 15 ml/min in a BSA aqueous solution of 5% by weight, which is adjusted to 37°C at a pH of 7.2, and in the case when the BSA has a sieving coefficient of 0.1 or more after a lapse of 30 minutes from the start of filtration, since the BSA is made in contact with the inner surface of the membrane, and since, after passing through the membrane, it is transferred while being also made in contact with the outer surface of the membrane, the pore inner surface and also outer surface of the membrane are defined as the surface area. In contrast, in the case when the sieving coefficient of the BSA is less than 0.1, since the BSA is hardly allowed to permeate the membrane, and is only made in contact with the membrane inner surface, the inner surface of the membrane is defined as the surface area.

The sieving coefficient of the BSA is calculated through the following expression:

$$(a) = 2 \times (b) / ((c) + (d))$$

Here, (a) indicates the sieving coefficient of the BSA, (b) indicates the BSA concentration of the filtered solution, (c) indicates the BSA concentration on the original solution side prior to the membrane separation, and (d) is the BSA concentration on the original solution side after the membrane separation.

With respect to the contact time, when it is too short, the operation might be finished prior to arrival at the adsorption equilibrium, and in contrast, when it is too long, the proteins might be denatured; therefore, it is set to 2 hours. Moreover, in the case when the amount of the BSA solution is too small, since the absolute amount of proteins becomes small, the amount thereof is set to 50 μl or more per 1 $cm^2$ of membrane area. Furthermore, since the amount of adsorption is greatly influenced by the temperature, the contacting process between the BSA solution and the substrate needs to be carried out at 25°C.

With respect to the quantifying process for the amount of adsorption of the adsorbed BSA, for example, the following method is proposed. After the substrate has been immersed in 50 ml of the PBS for 10 seconds two times and then washed, the resulting substrate is immersed in an acetic acid solution of 50 volume % in a range from 1 ml or more to 30 ml or less, at room temperature for 12 hours, and the acetic acid solution is then freeze-dried. The dried BSA is dissolved in an aqueous solution containing a blocking agent so that the dried BSA is not again adsorbed to the walls,

and the resulting solution is quantified. The quantifying process of the BSA in the solution can be conducted by using a Bovine Albumin ELISA Quantitation Kit (E10-113) made by BETHYL CO., or a device identical thereto.

When proteins are dissolved in water, a hydrophilic domain exists on the surface of the protein molecule, with a hydrophobic domain existing inside the protein. It is considered that when the protein is made in contact with a hydrophobic substrate, the inside hydrophobic domain is exposed to the surface, and adsorbed onto the substrate through a hydrophobic interaction. Therefore, in order to restrain the adsorption of the protein, it is effective to hydrophilize the substrate surface. In particular, it is preferable to graft a hydrophilic polymer on the substrate surface because a protein adsorption restraining effect, derived from an excluded volume effect due to micro-Brownian movements of a hydrophilic polymer chain extended into a protein solution, is expected, in addition to an effect of hydrophilization by the hydrophilic polymer.

The hydrophilizing treatment is a treatment used for adding hydrophilicity to the substrate surface, and a method in which hydrophilic polymer is grafted by irradiation with radioactive rays is selected. In particular, a method in which a hydrophilic polymer is grafted by using an irradiation with radioactive rays is employed, since this method easily controls the hydrophilicity by selecting the kind and molecular weight of the hydrophilic polymer, generates less by-products, and also simultaneously carries out a sterilizing process.

The hydrophilic functional group refers to a functional group that produces a weak bond with a water molecule by an electrostatic interaction, a hydrogen bond and the like. The hydroxyl group, which is a nonionic functional group, has a small interaction with proteins having a strong surface charge, and also has a small oxidation/reduction force so that the resulting denaturation of protein is small.

The hydrophilic polymer refers to a polymer which is soluble in water and a polymer which, even if it is insoluble in water, is allowed to exert a weak interaction with a water molecule through an electrostatic interaction or a hydrogen bond. In the present invention at least one portion of the hydrophilic polymer is polyvinyl alcohol or a copolymer of polyvinyl alcohol as polyvinyl alcohol or a polyvinyl alcohol copolymer exerts high effects. With respect to the copolymer of polyvinyl alcohol, such a copolymer in which the number of vinyl alcohol units that serve as the monomer repetitive units contained inmolecules constituting the copolymer is represented by 10% or more to less than 100%, with respect to the number of all the monomer repetitive units, is preferably used.

With respect to the polyvinyl alcohol copolymer, a copolymer, which includes a monomer repetitive unit indicated by a chemical structural formula (1) and a monomer repetitive unit indicated by a chemical structural formula (2) in its molecule, is preferably used. Moreover, another copolymer component may be contained therein. Here, the saponification degree refers to a numeric value obtained from an expression (1). When the saponification degree is low, the hydrophilicity tends to become low to cause a reduction in the dissolving property to water, resulting in a difficulty in applying it as an aqueous solution to the substrate so as to be surface treated; therefore, the saponification degree is preferably set to 0.70 or more, more preferably to 0.74 or more, most preferably, in a range from 0.78 or more to less than 1. When the saponification degree is 1, polyvinyl alcohol is prepared, and this is also used as a preferable mode as described earlier.

[Chemical Formula (1)]

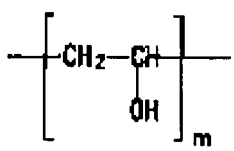

[Chemical Formula (2)]

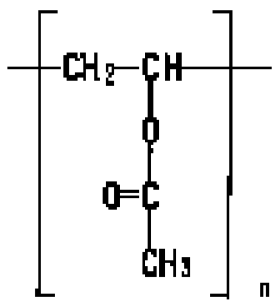

$$(k) = (m)/((n) + (m)) \quad \text{Expression (1)}$$

Symbols in (Expression 1) are explained as follows:

(k): Saponification degree
(m) : Number of monomer repetitive units indicated by formula (1) in polyvinyl alcohol copolymer
(n) : Number of monomer repetitive units indicated by formula (2) in polyvinyl alcohol copolymer

Additionally, the copolymer may be any one of a random copolymer, an alternate copolymer, a block copolymer and a graft copolymer, and may also be a combination of these.
With respect to the molecular weight of the hydrophilic polymer, since, when it is small, the molecular motility of the hydrophilic polymer becomes small, the weight-average molecular weight is preferably set to 1000 or more, more preferably, to 5000 or more, most preferably, to 10000 or more.
With respect to a method used for introducing the hydrophilic polymer to the substrate a grafting process onto the substrate is used since this causes less elution. In particular, a grafting process using radioactive rays is selected since this generates less by-products. Preferably, the substrate is made in contact with a hydrophilic polymer solution having a hydroxyl group, preferably an aqueous solution thereof, so that the grafting process is carried out by using radioactive rays.
With respect to the radioactive rays, $\alpha$-rays, $\beta$-rays, $\gamma$-rays, X-rays, ultraviolet rays, electron beams and the like may be used. In the case when a fractionation device is preserved for a long time, fungi might develop to cause degradation in performances. In particular, in the case of a fractionation device provided with separation or concentrating means, moisture can be preliminarily contained in these means; however, since the moisture-containing state causes a higher risk of developing fungi, a sterilizing process is preferably carried out. In recent years, from the viewpoint of easiness in use, a radioactive-ray sterilizing method has been used in many cases, and in particular, electromagnetic wave beams such as $\gamma$-rays and electron beams are desirably used. In other words, by adopting a method using radioactive rays, sterilizing and hydrophilizing processes can be desirably achieved simultaneously. The dose is preferably set to 15 kGy or more when the sterilizing process is required, and when no sterilizing process is required, it is preferably set in a range from 0.5 kGy or more to 200 kGy or less, more preferably, in a range from 1 kGy or more to 100 kGy or less, from the viewpoint of efficiency of hydrophilizing processes and prevention of degradation of the substrate.
In the fractionation device of the present invention, upon carrying out a separation or concentrating process by using a membrane, any of a flat film type separation membrane (hereinafter, referred to as "flat membrane"), such as a plane filter and a cartridge-type filter, a hollow-type separation membrane (hollow fiber membrane) such as hollow fibers may be used, and in general, since the hollow fibers have a larger membrane surface area per amount of the treatment solution, and make it possible to reduce the pressure loss, they can be used most efficiently. In order to increase the membrane surface area per amount of the treatment solution, the inner diameter of each hollow fiber is preferably made smaller, and is preferably set to 1000 $\mu$m or less, more preferably to 500 $\mu$m or less. Moreover, the plane filter has an advantage that a membrane forming process is easily carried out at low costs. For example, the material for the membrane is preferably at least one material selected from the group consisting of cellulose, cellulose acetate, polycarbonate, polysulfone, polymethacrylate such as polymethyl methacrylate, polyacrylate, polyamide, polyvinylidene fluoride, polyacrylonitrile, polyester, polyurethane, polystyrene, polyethylene and polypropylene and derivatives of these. Among these, polysulfone, which has been often used for a dialyzer in recent years, is a preferable material because of its superior fractionating property.

[Examples]

The following description will discuss the present invention in detail by means of examples.

<<Example A>>

<Measurements on amount of BSA adsorption in recovery container>

ALBUMIN, BOVINE (A-7906, Lot. 41k1270) (100 mg), made by SIGMA, was dissolved in 100 ml of PBS to prepare a BSA solution of 1000 $\mu$g/ml. To a recovery container 2 ml of the BSA solution was added, and this was capped so as to prevent the solution from evaporating, and put aside still at 25°C for four hours. At this time, the BSA solution is made in contact with an area of 4.0 $cm^2$ on the inner surface of the recovery container, and this area was used so as to calculate the amount of BSA adsorption. The BSA solution in the recovery container was removed by sucking it by

using an aspirator, and washed by 3 ml of PBS five times. To the recovery container was added 3 ml of an aqueous solution of acetic acid having 50 volume %, and this was put aside still at 25°C for 12 hours, and then freeze-dried together with the recovery container. In order to prevent BSA to be recovered from adsorbing onto the surface of the recovery container, 1 ml of a solution, prepared by dissolving 40 g of powder of Block Ace (UK-B40, lot. STK084206) made by DAINIPPON SUMITOMO PHARMA CO. , LTD. in 1 liter of water, was put into the recovery container, and this was mixed by a vortex mixer so that BSA adhered to the inner wall of the recovery container was dissolved to prepare a BSA quantifying analysis sample. With respect to the quantifying process of the BSA, an analysis was carried out by using a Bovine Albumin ELISA Quantitation Kit (E10-113, lotE10-113-11) made by BETHYL Co., in accordance with the instruction added to the Kit. The amount of adsorption was calculated based upon an expression (2).

$$A = B/C \quad \text{Expression (2)}$$

Symbols in the expression (2) are explained below:

A: Amount of BSA adsorption ($ng/cm^2$)
B: Amount of BSA obtained through BSA quantifying analysis (ng)
C: Contact area between BSA solution and recovery container ($cm^2$)

<Measurements on amount of BSA adsorption on hollow fiber membranes>

Thirty hollow fiber membranes used for a mini-module were bundled and both ends were fixed in a glass tube type module case with an epoxy type potting agent in a manner so as not to clog the hollow parts of the hollow fiber membranes so that a mini-module was prepared. The mini-module had an inner diameter of about 5 mm and a length of about 12 cm and two ports (blood ports) connected to the inside of each hollow fiber membrane, as well as two ports (dialysis ports) connected to the outside thereof, in the same manner as a common hollow fiber membrane type dialyzer. The hollow fiber membranes of the mini-module and the inside of the module were washed with distilled water. After that, PBS was injected thereto through the dialysis ports, and then capped so that the outside of the hollow fibers was filled with PBS. One of the blood ports was connected to a silicon tube with a Peri-Strat™ pump being attached in the middle of the tube. The other blood port was also connected to a silicon tube. The open ends of these two silicon tubes were inserted into a PP tube (188261) made by Greiner Bio-One Co., Ltd. To the PP tube was put 10 ml of a BSA solution of 1000 μg/ml prepared by dissolving ALBUMIN, BOVINE (A-7906, Lot. 41k1270) (100 mg) made by SIGMA , in 100 ml of PBS, and this was circulated at 25°C for 4 hours at a flow rate of 1 ml/min by the Peri-Strat™ pump. Thereafter, 300 ml of PBS was quickly transported at a flow rate of 10 ml/min by the Peri-Strat™ pump. At this time, the PBS discharged from the silicon tubes was discarded and was not circulated.

Thereafter, the PBS was removed from the inside of the mini-module. The mini-module was disassembled and the hollow fibers were taken out. The length of the hollow fibers thus taken out was 15 cm. The hollow fibers were cut into a length of 1 cm, and the entire amount thereof was put into a PP tube (188261) made by Greiner Bio-One Co. , Ltd. , and to this was added 5 ml of an aqueous solution of acetic acid of 50% by volume, and this was set aside still at 25°C for 12 hours. Thereafter, the hollow fibers were filtered and separated, and the acetic acid solution was freeze-dried together with the recovery container. To the recovery container was added 2 ml of a solution prepared by dissolving 40 g of powder of Block Ace (UK-B40, lot. STK084206) made by DAINIPPON SUMITOMO PHARMA CO. , LTD. in 1 liter of water, and this was mixed by a vortex mixer so that BSA adhered to the inner wall of the recovery container was dissolved to prepare a BSA quantifying analysis sample. With respect to the quantifying process of the BSA, an analysis was carried out by using a Bovine Albumin ELISA Quantitation Kit (E10-113, lotE10-113-11) made by BETHYL Co., in accordance with the instruction added to the Kit. The amount of adsorption was calculated based upon an expression (3). Moreover, the surface area of the inside of the hollow fibers was calculated based upon an expression (4).

$$D = E/F \quad \text{Expression (3)}$$

Symbols in the expression (3) are explained below:

D: Amount of BSA adsorption ($ng/cm^2$)
E: Amount of BSA obtained through BSA quantifying analysis (ng)
F: Surface area of the inside hollow fibers ($cm^2$)

$$F = (G/2)^2 P \cdot H \cdot I \quad \text{Expression (4)}$$

Symbols in the expression (4) are explained below:

F: Surface area of the inside hollow fibers ($cm^2$)
G: Inner diameter of hollow fibers
P: Ratio of circumference
H: Length of each of hollow fibers taken out of the module
I: Number of hollow fibers

<Measurements on β2 microglobulin in solution>

An analysis was carried out by using a β2-Microglobulin-EIA TEST (Code 305-11011), Grazime made by Wako Pure Chemical Industries, Ltd. in accordance with the instruction added to the kit.

<Measurements on albumin in serum and recovered solution>

An analysis was carried out by using a Human Albumin ELISA Quantitation Kit (E80-129, lotE80-129-9) made by BETHYL Co., in accordance with the instruction added to the Kit.

<Production of recovery container (1)>

Polyvinyl alcohol, made by Nakarai Tesque, Inc. (28211-25, (actually, copolymer of vinyl alcohol and vinyl acetate, saponification degree: 86.5 to 89.0 %), LotNo.M2B1968), was dissolved in ultrapure water to prepare an aqueous solution of a polyvinyl alcohol copolymer having a concentration of 1000 ppm. To a bag with a chuck (Unipack E-4) made by Seisan Nippon Ltd. was added 50 ml of the polyvinyl alcohol aqueous solution, and a polystyrene round tube (352054) of 5 ml made by BECTON DICKINSON CO. , LTD. was immersed therein, and this was irradiated with γ-rays. At this time, the dose of absorption of γ-rays was 26 kGy. After the irradiation with γ-rays, the polystyrene round tube was washed with 1 liter of ultrapure water, and dried by an oven at 50°C for 3 hours to obtain a recovery container (1).

<Preparation of recovery container (2)>

A polystyrene round tube (352054) of 5 ml made by BECTON DICKINSON CO., LTD., as it was, was used as a recovery container (2).

<Production of mini-module (1)>

The resin connecting portions of two ends of a module of a dialyzer BS1.8L (Lot. 20440312) made by Toray Industries, Inc. were cut to obtain a hollow fiber membrane. The size of the resulting hollow fiber membrane was 200 μm in inner diameter and 40 μm in thickness. One hundred of the hollow fiber membranes were bundled and both ends were fixed in a glass tube type module case with an epoxy type potting agent in a manner so as not to clog the hollow parts of the hollow fiber membranes so that a mini-module was prepared. The mini-module had an inner diameter of about 5 mm and a length of about 17 cm and two ports (blood ports) connected to the inside of each hollow fiber membrane as well as two ports (dialysis ports) connected to the outside thereof, in the same manner as a common hollow fiber membrane type dialyzer. The hollow fiber membranes of the mini-module and the inside of the module were washed with distilled water. After that, PBS was injected thereto so that a hollow fiber membrane mini-module (hereinafter, referred to as mini-module (1)) was obtained.

<Production of mini-module (2)>

Forty of the hollow fiber membranes, cut out from a dialyzer BS1.8L made by Toray Industries, Inc., were bundled and both ends were fixed in a glass tube type module case with an epoxy type potting agent in a manner so as not to clog the hollow parts of the hollow fiber membranes so that a mini-module was prepared. The mini-module had an inner diameter of about 5 mm and a length of about 17 cm and two ports (blood ports) connected to the inside of each hollow fiber membrane as well as two ports (dialysis ports) connected to the outside thereof, in the same manner as a common hollow fiber membrane type dialyzer. The hollow fiber membranes of the mini-module and the inside of

the module were washed with distilled water. After that, PBS was injected there to so that a hollow fiber membrane mini-module (hereinafter, referred to as mini-module (2)) was obtained.

<Production of mini-module (3)>

Polysulfone (UDEL® P-3500, manufactured by Solvay Advanced Polymers, L.L.C.)(18 parts by weight) and polyvinylpyrrolidone (K 30, manufactured by BASF Inc.) (9 parts by weight) were added to a mixed solvent of N, N' -dimethylacetamide (72 parts by weight) and water (1 part by weight) and heated at 90°C for 14 hours for dissolution to obtain a dope solution. The dope solution was jetted out of an outside tube of a tube-in orifice type spinneret having an outer diameter of 0.3 mm and an inner diameter of 0.2 mm. As a core solution, a solution containing N, N' -dimethylacetamide (58 parts by weight) and water (42 parts by weight) was jetted out of the inside tube.

The jetted dope solution was led to a coagulation bath of 100% water, after passing through a distance of 350 mm from the spinneret to reach the liquid surface of the coagulation bath, a hollow fiber membrane was obtained. The structure of the obtained hollow fiber membrane was observed by an electron microscope (S800, manufactured by Hitachi Ltd.) to find that the membrane had an asymmetric structure. Ten thousand hollow fiber membranes obtained were inserted in a cylindrical plastic case having a dialysis solution inlet and a dialysis solution outlet in the same manner as a common dialyzer, and both end parts were sealed with a resin to obtain a hollow fiber membrane module having an effective membrane surface area of 1. 6 $m^2$. After the hollow fiber membrane module was washed with water, hollow fiber membranes were cut out from the module, and 100 of these were prepared. These hollow fiber membranes were dried at 50°C and 13% relative humidity for 24 hours. Both terminal parts of the resulting hollow fiber membranes were fixed in a glass tube type module case with an epoxy type potting agent in the same manner as example A4 to produce a mini-module. The hollow fiber membranes of the mini-module and the inside of the module were washed with distilled water. Thereafter, PBS was injected thereto so that a hollow fiber membrane mini-module for concentration (hereinafter, referred to as mini-module (3)) was obtained.

<Production of mini-module (4)>

A mini-module was produced in the same manner as the mini-module (1). In this case, however, although the hollow fiber membranes of the mini-module and the inside of the module were washed with distilled water, the injection of BPS was not carried out.

Polyvinyl alcohol, made by Nakarai Tesque, Inc. (28311-25, LotNo.M2B1968), was dissolved in ultrapure water to prepare an aqueous solution of polyvinyl alcohol having a concentration of 1000 ppm, and 10 ml of this solution was injected to one of the blood ports inside the hollow fiber membrane of the mini-module at a flow rate of 1 ml/min by a Peri-Star™ pump, and discharged from the other blood port through the inside of the hollow fibers; then, the solution was injected into the dialyzer port on the blood port side through a tube, and discharged from the other dialyzer port.

Thereafter, $\gamma$-rays were radiated to the module in which the inside and the outside of the hollow fibers of the mini-module were filled with the polyvinyl alcohol, with the four ports being tightly plugged, and at this time, the dose of absorption of $\gamma$-rays was 26 kGy. The hollow fiber membranes of the mini-module and the inside of the module were washed with 3 liters of distilled water at 40°C.

Thereafter, PBS was injected thereto to obtain a hollow fiber membrane mini-module (hereinafter, referred to simply as mini-module (4)).

<Production of mini-module (5)>

A mini-module was produced in the same manner as the mini-module (2). In this case, however, although the hollow fiber membranes of the mini-module and the inside of the module were washed with distilled water, the injection of BPS was not carried out.

Polyvinyl alcohol, made by Nakarai Tesque, Inc. (28311-25, LotNo.M2B1968), was dissolved in ultrapure water to prepare an aqueous solution of polyvinyl alcohol having a concentration of 1000 ppm, and 10 ml of this solution was injected to one of the blood ports inside the hollow fiber membrane of the mini-module at a flow rate of 1 ml/min by a Peri-Star™ pump, and discharged from the other blood port through the inside of the hollow fibers; then, the solution was injected into the dialyzer port on the blood port side through a tube, and discharged from the other dialyzer port.

Thereafter, $\gamma$-rays were radiated to the module in which the inside and the outside of the hollow fibers of the mini-module were filled with the polyvinyl alcohol, with the four ports being tightly plugged, and at this time, the dose of absorption of $\gamma$-rays was 26 kGy. The hollow fiber membranes of the mini-module and the inside of the module were washed with 3 liters of distilled water at 40°C.

Thereafter, PBS was injected thereto to obtain a hollow fiber membrane mini-module (hereinafter, referred to simply as mini-module (5)).

<Production of mini-module (6)>

A mini-module was produced in the same manner as the mini-module (3). In this case, however, although the hollow fiber membranes of the mini-module and the inside of the module were washed with distilled water, the injection of BPS was not carried out.

Polyvinyl alcohol, made by Nakarai Tesque, Inc. (28311-25, LotNo.M2B1968), was dissolved in ultrapure water to prepare an aqueous solution of polyvinyl alcohol having a concentration of 1000 ppm, and 10 ml of this solution was injected to one of the blood ports inside the hollow fiber membrane of the mini-module at a flow rate of 1 ml/min by a Peri-Star™ pump, and discharged from the other blood port through the inside of the hollow fibers; then, the solution was injected into the dialyzer port on the blood port side through a tube, and discharged from the other dialyzer port. Thereafter, $\gamma$-rays were radiated to the module in which the inside and the outside of the hollow fibers of the mini-module were filled with the aqueous solution of polyvinyl alcohol, with the four ports being-tightly plugged, and at this time, the dose of absorption of $\gamma$-rays was 26 kGy. The hollow fiber membranes of the mini-module and the inside of the module were washed with 3 liters of distilled water at 40°C. Thereafter, PBS was injected thereto to obtain a hollow fiber membrane mini-module for concentration (hereinafter, referred to simply as mini-module (6)).

<Example A1>

First, one mini-module (1) was prepared, and one of the ports in the outside thereof was capped and the other port was connected via a silicone tube. On the other hand, with respect to a solution in the inside of the hollow fiber membranes, the raw solution inlet and the raw solution outlet of the module were connected with each other through a silicone tube to form a solution circulation channel and a Peri-Strat™ pump was attached to the channel so as to circulate the solution therein. Further, a three-way valve was installed in the middle of the solution circulation channel, and an injection pump was attached to one of the three-way valves. The resulting mini-module was used as a membrane separation unit in the first stage. Moreover, with respect to one mini-module of mini-modules (2), one of the ports in the outside thereof was capped and the other port was connected to a silicone tube. With respect to a solution in the inside of the hollow fiber membranes of the mini-module (2), the raw solution inlet and the raw solution outlet of the module were connected with each other through a silicone tube to form a solution circulation channel and a Peri-Strat™ pump was attached to the channel so as to circulate the solution therein. Further, a three-way valve was installed in the middle of the solution circulation channel. Thus, a membrane separation unit in the second stage was prepared. Moreover, with respect to the other mini-module (2), one of the ports in the outside thereof was capped and the other port was connected to a silicone tube. With respect to a solution in the inside of the hollow fiber membranes of the second mini-module (2) also, the raw solution inlet and the raw solution outlet of the module were connected with each other through a silicone tube to form a solution circulation channel and a Peri-Strat™ pump was attached in the middle of the channel so as to circulate the solution therein. Further, a three-way valve was installed in the middle of the solution circulation channel. The resulting mini-module was used as a membrane separation unit in the third stage.

One of the ports in the outside of a mini-module (3) was capped and the other port was used as a filtrate outlet. With respect to a solution in the inside of the hollow fiber membranes of the mini-module (3), the raw solution inlet and the raw solution outlet of the module were connected with each other through a silicone tube to form a solution circulation channel so that a Peri-Strat™ pump was used so as to circulate the raw solution therein. Further, two three-way valves were installed in the middle of the solution circulation channel. Thus, a concentration membrane unit was prepared.

The treated solution recovery port of the mini-module of the separation membrane unit in the third stage and the three-way valve of the concentration membrane unit were connected with each other with a silicone tube. Moreover, a treated solution recovery port of the concentration unit constituted by a three-way valve was installed in the middle of the solution circulation channel of the concentration membrane unit so that during a concentration operation, only the solution circulation channel was allowed to open. A recovery container (1) was attached to the front side of the treated solution recovery port. Thus, the entire body of the system was filled with PBS to produce a compounded system including the membrane separation unit for fractionating proteins with a molecular weight equal to or higher than that of albumin by molecular sieving and the unit for concentrating proteins bonded directly to the separation unit.

Fig. 1 shows a schematic view of the separation system used in example A1. The solution flow is shown as arrows. Serum and a diluted solution (PBS) are injected into the solution circulation channel 102 in the first stage by the injection pump 100 via the three-way valve 101. The solution is sent further by the pump 103 in the first stage, and injected into the first separation membrane module 105 (the mini-module (1)), and circulated in the solution circulation channel 102 in the first stage. The solution treated in the first membrane separation unit is obtained through the treated solution recovery port 104 in the first stage of the membrane separation unit. Next, the solution is injected into the solution circulation channel 202 in the second stage via the three-way valve 201 of the membrane separation unit in the second stage, and further sent by the pump 203 in the second stage and injected into the second separation membrane module 205 (the first mini-module (2)) so as to be circulated in the solution circulation channel 202 in the second stage. The

solution treated in the second membrane separation unit is obtained through the treated solution recovery port 204 of the membrane separation unit in the second stage. Moreover, the solution is further injected into the solution circulation channel 302 in the third stage by the pump 303 in the third stage via the three-way valve 301 of the membrane separation unit in the third stage, and then sent and injected into the third separation membrane module 305 (the second mini-module (2)) so as to be circulated in the solution circulation channel 302. The solution treated in the third membrane separation unit is obtained through the treated solution recovery port 304 of the membrane separation unit in the third stage. Furthermore, the treated solution is injected into the solution circulation channel 402 in the fourth stage by the pump 403 in the fourth stage via the three-way valve 401 of the concentration membrane unit, and further sent and injected into the concentration membrane module 405 (mini-module (3)) so as to be circulated in the solution circulation channel 402 in the fourth stage. In this case, the solution filtered through the concentration membrane module 405 is taken out of the filtrate outlet port 404 of the concentration membrane unit, and discarded. After completion of the filtering and concentrating operations, the solution remaining in the solution circulation channel 402 in the fourth stage of the mini-module (3) is taken out into the recovery container 407 (recovery container (1)) by opening the three-way valve 406 of the concentration unit.

Practical conditions were as follows. After 1 mL of human serum (H1388 (Lot 073K445), made by Sigma) was added at 0.2 mL/min to the membrane separation unit in the first stage, it was circulated at a flow rate of 5.0 mL/min in common in the respective solution circulation channels of the first-stage membrane separation unit, the second-stage membrane separation unit, and the third-stage membrane separation unit so that a filtration process was carried out at a filtrate flow rate of 0.2 mL/min in common in the respective modules and 20°C for 4 hours. At that time, the amount of the circulated solution in the respective separation units and the concentration unit was kept constant by adding PBS in an amount equivalent to that of the filtered solution at 0.2 mL/min by the injection pump 100.

The concentration of albumin of the solution obtained in the recovery container 407 (recovery container (1)) after 4-hour operation was 0.336 μg and the concentration of β2-microglobulin was 0.853 μg, and with respect to the human serum used as the raw solution, the concentration of albumin was 31200 μg and the concentration of β2-microglobulin was 1.19 μg; thus, it becomes possible to remove a considerable amount of albumin, while the concentration of β2-microglobulin is properly maintained.

When the amount of BSA adsorption was found separately, it was 16.6 ng/cm$^2$ in the recovery container (1), 397.9 ng/cm$^2$ in the hollow fibers of the mini-module (1), 378.4 ng/cm$^2$ in the hollow fibers of the mini-module (2), and 429.3 ng/cm$^2$ in the hollow fibers of the mini-module (3).

<Example A2>

First, one mini-module (4) was prepared, and one of the ports in the outside thereof was capped and the other port was connected via a silicone tube. On the other hand, with respect to a solution in the inside of the hollow fiber membranes, the raw solution inlet and the raw solution outlet of the module were connected with each other through a silicone tube to form a solution circulation channel and a Peri-Strat™ pump was attached to the channel so as to circulate the solution therein. Further, a three-way valve was installed in the middle of the solution circulation channel, and an injection pump was attached to one of the three-way valves. The resulting mini-module was used as a membrane separation unit in the first stage. Moreover, with respect to one mini-module of mini-modules (5), one of the ports in the outside thereof was capped and the other port was connected to a silicone tube. With respect to a solution in the inside of the hollow fiber membranes of the mini-module (5), the raw solution inlet and the raw solution outlet of the module were connected with each other through a silicone tube to form a solution circulation channel and a Peri-Strat™ pump was attached to the channel so as to circulate the solution therein. Further, a three-way valve was installed in the middle of the solution circulation channel. Thus, a membrane separation unit in the second stage was prepared. Moreover, with respect to the other mini-module (5), one of the ports in the outside thereof was capped and the other port was connected to a silicone tube. With respect to a solution in the inside of the hollow fiber membranes of the second mini-module (5) also, the raw solution inlet and the raw solution outlet of the module were connected with each other through a silicone tube to form a solution circulation channel and a Peri-Strat™ pump was attached in the middle of the channel so as to circulate the solution therein. Further, a three-way valve was installed in the middle of the solution circulation channel. The resulting mini-module was used as a membrane separation unit in the third stage.

One of the ports in the outside of a mini-module (6) was capped and the other port was used as a filtrate outlet. With respect to a solution in the inside of the hollow fiber membranes of the mini-module (6), the raw solution inlet and the raw solution outlet of the module were connected with each other through a silicone tube to form a solution circulation channel so that a Peri-Strat™ pump was used so as to circulate the raw solution therein. Further, two three-way valves were installed in the middle of the solution circulation channel. Thus, a concentration membrane unit was prepared.

The treated solution recovery port of the mini-module of the separation membrane unit in the third stage and the three-way valve of the concentration membrane unit were connected with each other with a silicone tube. Moreover, a treated solution recovery port of the concentration unit constituted by a three-way valve was installed in the middle of

the solution circulation channel of the concentration membrane unit so that during a concentration operation, only the solution circulation channel was allowed to open. A recovery container (1) was attached to the front side of the treated solution recovery port. Thus, the entire body of the system was filled with PBS to produce a compounded system including the membrane separation unit for fractionating proteins with a molecular weight equal to or higher than that of albumin by molecular sieving and the unit for concentrating proteins bonded directly to the separation unit, in the same manner as example 1.

The injection, separation, concentration and recovery operations were carried out in the same manner as example 1. Practical conditions were as follows. After 1 mL of serum was added at 0.2 mL/min to the membrane separation unit in the first stage, it was circulated at a flow rate of 5.0 mL/min in common in the respective solution circulation channels of the first-stage membrane separation unit, the second-stage membrane separation unit, and the third-stage membrane separation unit so that a filtration process was carried out at a filtrate flow rate of 0.2 mL/min in common in the respective modules and 20°C for 4 hours. At that time, the amount of the circulated solution in the respective separation units and the concentration unit was kept constant by adding PBS in an amount equivalent to that of the filtered solution at 0.2 mL/min by the injection pump.

The concentration of albumin of the solution obtained in the recovery container (1) after 4-hour operation was 0.251 μg and the concentration of β2-microglobulin was 0.981 μg, and with respect to the human serum used as the raw solution, the concentration of albumin was 31200 μg and the concentration of β2-microglobulin was 1.19 μg; thus, it becomes possible to remove a considerable amount of albumin, while the concentration of β2-microglobulin is properly maintained.

When the amount of BSA adsorption was found separately, it was 17.6 ng/$cm^2$ in the recovery container (1), 35.6 ng/$cm^2$ in the hollow fibers of the mini-module (4), 39.2 ng/$cm^2$ in the hollow fibers of the mini-module (5), and 42.8 ng/$cm^2$ in the hollow fibers of the mini-module (6).

<Comparative Example A1>

A filtration operation was carried out for four hours in the same manner as example A1 except that in place of the recovery container (1) in example A1, the recovery container (2) was used. The concentration of albumin of the solution obtained in the recovery container (2) was 0.490 μg and the concentration of β2-microglobulin was 0.202 μg, and with respect to the human serum used as the raw solution, the concentration of albumin was 31200 μg and the concentration of β2-microglobulin was 1.19 μg; although it was possible to remove albumin, the recovery rate of β2-microglobulin was low.

When the amount of BSA adsorption was found separately, it was 236.4 ng/$cm^2$ in the recovery container (2), 377.1 μg/$cm^2$ in the hollow fibers of the mini-module (1), 394.5 ng/$cm^2$ in the hollow fibers of the mini-module (2), and 429.3 ng/$cm^2$ in the hollow fibers of the mini-module (3).

The results are shown in Tables 1 and 2 collectively. Table 1 indicates the amount of BSA adsorption, and Table 2 indicates the amount of recovery of β2-microglobulin. In examples A1 and A2 where the recovery container or the hollow fibers having an amount of adsorption of 50 ng/$cm^2$. or less was used, the amount of recovery of β2-microglobulin was high; in contrast, in comparative example A1 where the recovery container and the hollow fiber membrane having an amount of adsorption of 50 ng/$cm^2$ or more were used, the amount of recovery of β2-microglobulin was low. As described-above, in a device in which a surface having a low amount of adsorption was partially introduced, a high recovery rate of β2-microglobulin was achieved. It is found that the deposition and clogging of albumin onto the membrane surface were suppressed, with the result that β2-microglobulin was preferably allowed to pass through the membrane and the adsorption loss of the filtered β2-microglobulin was also suppressed, making it possible to provide a high recovery rate.

<<Example B>>

With respect to a polystyrene test tube ("5 ml Polystyrene Round-Bottom Tube", made by BECTON DICKINSON CO., LTD.) and a polypropylene test tube ("5 ml Polypropylene Round-BottomTube", made by BECTON DICKINSON CO., LTD.) used in the fractionation device of the present invention so as to recover the solution containing fractionated trace proteins, adsorption and recovery performances were compared.

<Adsorption test of human β2-microglobulin>

With respect to the adsorption evaluation of proteins onto a substrate surface, the following description will discuss a test in which a human β2-microglobulin solution is used. The human β2-microglobulin (hereinafter, referred to simply as β2-MG), used in the examples, corresponds to Recombinant Human β2-microglobulin, Cat. Code 47194000, made by ORIENTAL YEAST CO., LTD, or a product identical to this. Moreover, the bovine serum albumin in the present

invention corresponds to Bovine Albumin Powder, Product Number A7906, made by SIGMA, or a product identical to this.

An aqueous solution of PBS (Dulbecco PBS, manufactured by NISSUI PHARMACEUTICAL CO., LTD) (hereinafter, referred to simply as PBS aqueous solution), which had been adjusted to contain 200 ng/ml of β2-MG (Recombinant Human β2-microglobulin, Cat. Code 47194000, made by ORIENTAL YEAST CO., LTD.) and 10 μg/ml of bovine serum albumin (Bovine Albumin Powder, Product Number A7906, made by SIGMA), was used as a protein solution (hereinafter, referred to as protein solution A). Since the proteins in the protein solution A are also adsorbed to a container used for the preparation, the adsorption test should be started within 5 minutes after the preparation. The protein solution A prepared as described above was used for the absorption test in the following manner. In other words, as a control sample, to a container (Safe lock tube, product No. 0030 120,086, made by Eppendorf Co., Ltd.) in which 100 μl of a blocking agent (Block Ace Cat. Code UK-B25, sold by DAINIPPON SUMITOMO PHARMA) had been put was added 500 μl of the protein solution A, and this was freeze-dried and stored at -70°C until just before the β2-MG concentration measurement. As an adsorption evaluation sample, to a container (Safe lock tube, product No. 0030 120,086, made by Eppendorf Co., Ltd.) which had been made in contact with a substrate to be evaluated and set aside still at 25°C for one hour and in which 100 μl of a blocking agent (Block Ace Cat. Code UK-B25, sold by DAINIPPON SUMITOMO PHARMA) had been put was added 500 μl of the protein solution A, and this was freeze-dried and stored at -70°C until just before the β2-MG concentration measurement. Here, the amount of addition of the protein solution A with respect to the substrate surface area to be evaluated was set in a range from 2 ml/$cm^2$ or more to 8 ml/$cm^2$ or less.

The measurement on the β2-mug concentration was carried out by using a β2-MG measuring kit (Grazime β2-microglobulin EIA TEST, Code. 305-11011, available from Wako Pure Chemical Industries, Ltd.) in accordance with the manual attached to the kit. The amount of adsorption of protein was calculated based upon (expression 5).

$$(a) = ((b) - (c))/(d) \quad \text{(Expression 5)}$$

Symbols in (expression 5) are explained below:

(a): Amount of adsorption of protein of plastic test tube (ng/$cm^2$)
(b): Amount of β2-MG (ng) in control sample
(c) : Amount of β2-MG (ng) in adsorption evaluation sample
(d) : Contact area ($cm^2$) between plastic test tube and protein solution A

<Example B1>

Apolystyrene test tube ("5 ml Polystyrene Round-BottomTube" made by BECTON DICKINSON CO., LTD.) was immersed in 100 ml of a 1000 ppm aqueous solution of vinyl alcohol-vinyl acetate copolymer (weight average molecular weight: 10000, saponification degree: 80%, Cat. No. 360627, made by Sigma-Aldrich Japan), and this was irradiated with γ-rays. At this time, the dose of absorption of γ-rays was 25 kGy. The polystyrene test tube was taken out of the polyvinyl alcohol aqueous solution, and washed with 500 ml of flowing water, and dried by an oven at 70°C for one hour. This test tube was used for the human β2-microglobulin adsorption test. The results are shown in Table 3.

<Example B2>

The same polystyrene test tube as the test tube first prepared in example B1 was immersed in 100 ml of a 10 ppm aqueous solution of polyvinyl alcohol-vinyl acetate copolymer of example B1, and this was irradiated with γ-rays. At this time, the dose of absorption of γ-rays was 26 kGy. The polystyrene test tube was taken out of the polyvinyl alcohol aqueous solution, and washed with 500 ml of flowing water, and dried by an oven at 70°C for one hour. This test tube was used for the human β2-microglobulin adsorption test. The results are shown in Table 3.

<Example B3>

The same polystyrene test tube as the test tube first prepared in example B1 was immersed in 100 ml of a 1000 ppm aqueous solution of polyvinyl alcohol (molecular weight: 22000, CodeNo. 28311-25, made by Nakarai Tesque, Inc.), and this was irradiated with γ-rays. At this time, the dose of absorption of γ-ray was 25 kGy. The polystyrene test tube was taken out of the polyvinyl alcohol aqueous solution, and washed with 500 ml of flowing water, and dried by an oven at 70°C for one hour. This test tube was used for the human β2-microglobulin adsorption test. The results are shown in Table 3.

<Example B4>

The same polystyrene test tube as the test tube first prepared in example B1 was immersed in 100 ml of a 10 ppm aqueous solution of the same polyvinyl alcohol as that used in example B3, and this was irradiated with γ-rays. At this time, the dose of absorption of γ-rays was 27 kGy. The polystyrene test tube was taken out of the polyvinyl alcohol aqueous solution, and washed with 500 ml of flowing water, and dried by an oven at 70°C for one hour. This test tube was used for the human β2-microglobulin adsorption test. The results are shown in Table 3.

<Example B5>

A polypropylene test tube ("5 ml Polystyrene Round-Bottom Tube" made by BECTON DICKINSON CO., LTD.) was immersed in 100 ml of a 1000 ppm aqueous solution of polyvinyl alcohol (molecular weight: 10000, Cat. No. 360627, made by Sigma-Aldrich Japan), and this was irradiated with γ-rays. At this time, the dose of absorption of γ-rays-was 25 kGy. The polystyrene test tube was taken out of the polyvinyl alcohol aqueous solution, and washed with 500 ml of flowing water, and dried by an oven at 70°C for one hour. This test tube was used for the human β2-microglobulin adsorption test. The results are shown in Table 3.

<Example B6>

The same polypropylene test tube as the test tube first prepared in example B5 was immersed in 100 ml of a 10 ppm aqueous solution of the same polyvinyl alcohol as that used in example B5, and this was irradiated with γ-ray. At this time, the dose of absorption of γ-rays was 25 kGy. The polystyrene test tube was taken out of the polyvinyl alcohol aqueous solution, and washed with 500 ml of flowing water, and dried by an oven at 70°C for one hour. This test tube was used for the human β2-microglobulin adsorption test. The results are shown in Table 3.

<Example B7>

The same polypropylene test tube as the test tube first prepared in example B5 was immersed in 100 ml of a 1000 ppm aqueous solution of polyvinyl alcohol (molecular weight: 22000, Code No. 28311-25, made by Nakarai Tesque, Inc.), and this was irradiated with γ-rays. At this time, the dose of absorption of γ-rays was 26 kGy. The polystyrene test tube was taken out of the polyvinyl alcohol aqueous solution, and washed with 500 ml of flowing water, and dried by an oven at 70°C for one hour. This test tube was used for the human β2-microglobulin adsorption test. The results are shown in Table 3.

<Example B8>

The same polypropylene test tube as the test tube first prepared in example B5 was immersed in 100 ml of a 10 ppm aqueous solution of the same polyvinyl alcohol as that used in example B7, and this was irradiated with γ-rays. At this time, the dose of absorption of γ-rays was 27 kGy. The polystyrene test tube was taken out of the polyvinyl alcohol aqueous solution, and washed with 500 ml of flowing water, and dried by an oven at 70°C for one hour. This test tube was used for the human β2-microglobulin adsorption test. The results are shown in Table 3.

<Comparative Example B1>

The same polystyrene test tube as the test tube first prepared in example B1 was washed with 500 ml of flowing water, and dried by an oven at 70°C for one hour. This test tube was used for the human β2-microglobulin adsorption test. The results are shown in Table 3.

<Comparative Example B2>

The same polypropylene test tube as the test tube first prepared in example B5 was washed with 500 ml of flowing water, and dried by an oven at 70°C for one hour. This test tube was used for the human β2-microglobulin adsorption test. The results are shown in Table 3.

<Comparative Example B3>

The same polystyrene test tube as the test tube first prepared in example B1 was immersed in 100 ml of a 1000 ppm aqueous solution of polyvinyl pyrrolidone (molecular weight: 50000, Kollidon 25, made by BASF), and this

was irradiated with γ-rays. At this time, the dose of absorption of γ-rays was 25 kGy. The polystyrene test tube was taken out of the polyvinyl pyrrolidone aqueous solution, and washed with 500 ml of flowing water, and dried by an oven at 70°C for one hour. This test tube was used for the human β2-microglobulin adsorption test. The results are shown in Table 3.

<Comparative Example B4>

The same polystyrene test tube as the test tube first prepared in example B1 was immersed in 100 ml of a 1000 ppm aqueous solution of polyvinyl pyrrolidone (molecular weight: 3000, Kollidon 12PF, made by BASF), and this was irradiated with γ-rays. At this time, the dose of absorption of γ-rays was 27 kGy. The polystyrene test tube was taken out of the polyvinyl alcohol aqueous solution, and washed with 500 ml of flowing water, and dried by an oven at 70°C for one hour. This test tube was used for the human β2-microglobulin adsorption test. The results are shown in Table 3.

<Comparative Example B5>

The same polystyrene test tube as the test tube first prepared in example B1 was immersed in 100 ml of a 1000 ppm aqueous solution of polyethylene glycol (molecular weight: 20000, Polyethylene Glycol 20000, made by Katayama Chemical Co., Ltd.), and this was irradiated with γ-rays . At this time, the dose of absorption of γ-rays was 25 kGy. The-polystyrene test tube was taken out of the polyvinyl alcohol aqueous solution, and washed with 500 ml of flowing water, and dried by an oven at 70°C for one hour. This test tube was used for the human β2-microglobulin adsorption test. The results are shown in Table 3.

<Comparative Example B6>

The same polystyrene test tube as the test tube first prepared in example B1 was immersed in 100 ml of a 1000 ppm aqueous solution of polyethylene glycol (molecular weight: 2000, Polyethylene Glycol 2000, made by Wako Pure Chemical Industries, Ltd.), and this was irradiated with γ-rays. At this time, the dose of absorption of γ-rays was 26 kGy. The polystyrene test tube was taken out of the polyvinyl alcohol aqueous solution, and washed with 500 ml of flowing water, and dried by an oven at 70°C for one hour. This test tube was used for the human β2-microglobulin adsorption test. The results are shown in Table 3.

<Comparative Example B7>

The same polystyrene test tube as the test tube first prepared in example B1 was immersed in 100 ml of a 1000 ppm aqueous solution of polyethylene imine (molecular weight: 25000, Cat. Code 40872-7, made by Sigma-Aldrich Japan), and this was irradiated with γ-rays. At this time, the dose of absorption of γ-rays was 26 kGy. The polystyrene test tube was taken out of the polyvinyl alcohol aqueous solution, and washed with 500 ml of flowing water, and dried by an oven at 70°C for one hour. This test tube was used for the human β2-microglobulin adsorption test. The results are shown in Table 3.

<Comparative Example B8>

The same polystyrene test tube as the test tube first prepared in example B1 was washed with 15 ml of methanol in its inside wall, and dried by an oven at 70°C for one hour. This test tube was immersed in 100 ml of a methanol solution containing 25000 ppm of a copolymer of MPC and butyl methacrylate for one minute. The test tube was taken out of a polyhydroxyethyl methacrylate solution, and the solution inside the test tube was discharged, and this was dried by an oven at 70°C for one hour. The test tube was washed with 500 ml of flowing water, and dried by an oven at 70°C for one hour. This test tube was used for the human β2-microglobulin adsorption test. The results are shown in Table 3.

<Comparative Example B9>

The same polypropylene test tube as the test tube first prepared in example B5 was washed with 15 ml of methanol in its inside wall, and dried by an oven at 70°C for one hour. This test tube was immersed in 100 ml of a methanol solution containing 25000 ppm of a copolymer of MPC and butyl methacrylate for one minute. The test tube was taken out of a polyhydroxyethyl methacrylate solution, and the solution inside the test tube was discharged, and this was dried by an oven at 70°C for one hour. The test tube was washed with 500 ml of flowing water, and dried by an oven at 70°C for one hour. This test tube was used for the human β2-microglobulin adsorption test. The results are shown in Table 3.

As clearly indicated by Table 3, as the results of the human β2-microglobulin adsorption test (in which a protein aqueous solution consisting of human β2-microglobulin having a concentration of 200 ng/ml and bovine serum albumin having a concentration of 10 μg/ml was used), the tests of the present invention (examples B1 to B8) made the amount of β2-MG adsorption (amount of human β2-microglobulin adsorption) smaller in comparison with comparative examples B1 to B9; therefore, the present invention makes it possible to effectively restrain the adsorption of trace biological components, and consequently to achieve a high recovery rate.

Table 1. Amount of BSA adsorption

| | Recovery container | Hollow fiber | | |
|---|---|---|---|---|
| Example A1 | Recovery container (1) 16.6ng/cm$^2$ | Mini-module(1)397. 9ng/cm$^2$ | Mini-module (2) 378. 4ng/cm$^2$ | Mini-module (3) 429. 3ng/cm$^2$ |
| Example A2 | Recovery container (1) 17.6ng/cm$^2$ | Mini-module (4) 35. 6ng/cm$^2$ | Mini-module (5) 39. 2ng/cm$^2$ | Mini-module (6) 42. 8ng/cm$^2$ |
| Comparative example A1 | Recovery container (2) 236.4ng/cm$^2$ | Mini-module(1)377. lng/cm$^2$ | Mini-module (2) 394. 5ng/cm$^2$ | Mini-module (3) 429. 3ng/cm$^2$ |

Table 2. Amount of albumin and β2-microglobulin before and after fractionation

| | Albumin | | β2-microglobulin | |
|---|---|---|---|---|
| | before | after | before | after |
| Example A1 | 31200 μg | 0.336 μg | 1.19 μg | 0.853 μg |
| Example A2 | 31200 μg | 0.251 μg | 1.19 μg | 0.981 μg |
| Comparative example A1 | 31200 μg | 0.490 μg | 1.19 μg | 0.202 μg |

Table 3.

| | Amount of β2-MG adsorption (ng/cm$^2$) |
|---|---|
| Example B1 | 1.03 |
| Example B2 | 1.23 |
| Example B3 | 1.21 |
| Example B4 | 2.05 |
| Example B5 | 1.64 |
| Example B6 | 1.63 |
| Example B7 | 1.03 |
| Example B8 | 1.23 |
| Comparative example B1 | 5.13 |
| Comparative example B2 | 8.61 |
| Comparative example B3 | 4.17 |
| Comparative example B4 | 4.01 |
| Comparative example B5 | 4.91 |
| Comparative example B6 | 4.94 |
| Comparative example B7 | 4.09 |
| Comparative example B8 | 6.35 |
| Comparative example B9 | 6.38 |

INDUSTRIAL APPLICABILITY

The fractionation device of the present invention is extremely effective for preparing a sample upon conducting a proteome analysis, and applied to the medical field, in particular, to find human diseases.

## Claims

1. A fractionation device used for proteins and/or peptides, comprising:

   a substrate having a surface with which proteins and/or peptides are made in contact,
   wherein the substrate has at least one portion of the surface where a hydrophilic polymer is grafted thereon by contacting the surface with an aqueous hydrophilic polymer solution having a hydroxyl group and by irradiating the surface with radioactive rays,
   and wherein at least one portion of the hydrophilic polymer is polyvinyl alcohol or a copolymer of polyvinyl alcohol, such that the surface adsorbs bovine serum albumin in an amount of 50 ng/$cm^2$ or less with respect to the substrate surface, when a bovine serum albumin solution of 1000 $\mu$g/ml is made in contact therewith.

2. The fractionation device as claimed in claim 1, wherein the hydrophilic polymer is polyvinyl alcohol.

3. The fractionation device as claimed in claim 1, wherein the copolymer of polyvinyl alcohol is a polyvinyl alcohol-polyvinyl acetate copolymer.

4. The fractionation device as claimed in claim 3, wherein the polyvinyl alcohol-polyvinyl acetate copolymer has a saponification degree in a range from 0.70 or more to less than 1.

5. The fractionation device as claimed in claim 1, wherein the substrate in which the amount of adsorption of bovine serum albumin is 50 ng/$cm^2$ or less is a membrane.

6. The fractionation device as claimed in claim 5, wherein the membrane has a shape of hollow fibers.

7. The fractionation device as claimed in claim 6, wherein the substrate is one of the following:

   a means for supplying a solution containing proteins and/or peptides;
   a means for separating proteins and/or peptides from the solution; and
   a means for concentrating proteins and/or peptides in the solution.

8. The fractionation device as claimed in claim 7, which serves as a pre-treatment device used for preparing a sample for a mass analysis on proteins and/or peptides.

9. The fractionation device as claimed in claim 8, wherein the proteins and/or peptides are a fluid derived from blood, such as blood, serum and plasma, urine, ascites, saliva, tear fluid, aqueous humor, cerebrospinal fluid, amniotic fluid, pleural fluid, or a fluid contained in cell extract.

10. A fractionation device used for proteins and/or peptides, comprising:

    a substrate having a surface with which proteins and/or peptides are made in contact,
    wherein the substrate has at least one portion of the substrate surface where a hydrophilic polymer is grafted thereon by contacting the surface with an aqueous hydrophilic polymer solution having a hydroxyl group and by irradiating the surface with radioactive rays,
    and wherein at least one portion of the hydrophilic polymer is polyvinyl alcohol or a copolymer of polyvinyl alcohol, such that the surface adsorbs human $\beta$2-microglobulin in an amount of 3 ng/$cm^2$ or less when a protein aqueous solution consisting of human $\beta$2-microglobulin having a concentration of 200 ng/ml and bovine serum albumin having a concentration of 10 $\mu$g/ml is made in contact with the substrate surface.

11. The fractionation device as claimed in claim 10, wherein the hydrophilic polymer is polyvinyl alcohol.

12. The fractionation device as claimed in claim 10, wherein the copolymer of polyvinyl alcohol is a polyvinyl alcohol-polyvinyl acetate copolymer.

**13.** The fractionation device as claimed in claim 12, wherein the polyvinyl alcohol-polyvinyl acetate copolymer has a saponification degree in a range from 0.70 or more to less than 1.

**14.** The fractionation device as claimed in claim 10, wherein the substrate in which the amount of adsorption of human β2-microglobulin is 3 ng/cm$^2$ or less comprises a membrane.

**15.** The fractionation device as claimed in claim 14, wherein the membrane has a shape of hollow fibers.

**16.** The fractionation device as claimed in claim 14, wherein the substrate is one of the following:

a means for supplying a solution containing proteins and/or peptides;
a means for separating proteins and/or peptides from the solution; and
a means for concentrating proteins and/or peptides in the solution.

**17.** The fractionation device as claimed in claim 16, which serves as a pre-treatment device used for preparing a sample for a mass analysis on proteins and/or peptides.

**18.** The fractionation device as claimed in claim 17, wherein the proteins and/or peptides are a fluid derived from blood, such as blood, serum and plasma, urine, ascites, saliva, tear fluid, aqueous humor, cerebrospinal fluid, amniotic fluid, pleural fluid, or a fluid contained in cell extract.

**19.** A fractionation device which is used for proteins and/or peptides, comprising:

a means for supplying a solution containing proteins and/or peptides;
a means for separating proteins and/or peptides from the solution; and
a means for concentrating proteins and/or peptides in the solution,
wherein at least one portion of the surface of the fractionation device with which proteins and/or peptides are made in contact is grafted with hydrophilic polymer by contacting the surface with an aqueous hydrophilic polymer solution having a hydroxyl group and by irradiating the surface with radioactive rays and the hydrophilic polymer is any one of polyvinyl alcohol, copolymers thereof and graft polymers thereof.

## Patentansprüche

**1.** Fraktionierungsvorrichtung, die für Proteine und/oder Peptide verwendet wird, umfassend:

ein Substrat, das eine Oberfläche aufweist, mit der Proteine und/oder Peptide in Kontakt gebracht werden,
worin das Substrat zumindest einen Teil der Oberfläche aufweist, auf den ein hydrophiles Polymer aufgepfropft wird, indem die Oberfläche mit einer wässrigen Lösung eines hydrophilen Polymers, das eine Hydroxylgruppe aufweist, in Kontakt gebracht wird und indem die Oberfläche mit radioaktiven Strahlen bestrahlt wird,
und worin zumindest ein Teil des hydrophilen Polymers Polyvinylalkohol oder ein Copolymer von Polyvinylalkohol ist, sodass die Oberfläche Rinderserumalbumin in einer Menge von 50 ng/cm$^2$ oder weniger in Bezug auf die Substratoberfläche adsorbiert, wenn eine Rinderserumalbuminlösung von 1.000 μg/ml damit in Kontakt gebracht wird.

**2.** Fraktionierungsvorrichtung nach Anspruch 1, worin das hydrophile Polymer Polyvinylalkohol ist.

**3.** Fraktionierungsvorrichtung nach Anspruch 1, worin das Copolymer von Polyvinylalkohol ein Polyvinylalkohol-Polyvinylacetat-Copolymer ist.

**4.** Fraktionierungsvorrichtung nach Anspruch 3, worin das Polyvinylalkohol-Polyvinylacetat-Copolymer einen Verseifungsgrad in einem Bereich von 0,70 oder mehr bis weniger als 1 aufweist.

**5.** Fraktionierungsvorrichtung nach Anspruch 1, worin das Substrat, in dem die Adsorptionsmenge von Rinderserumalbumin 50 ng/cm$^2$ oder weniger beträgt, eine Membran ist.

**6.** Fraktionierungsvorrichtung nach Anspruch 5, worin die Membran eine Form von Hohlfasern aufweist.

**7.** Fraktionierungsvorrichtung nach Anspruch 6, worin das Substrat eines der folgenden ist:

ein Mittel zum Bereitstellen einer Lösung, die Proteine und/oder Peptide enthält;
ein Mittel zum Abtrennen von Proteinen und/oder Peptiden von der Lösung; und
ein Mittel zum Konzentrieren von Proteinen und/oder Peptiden in der Lösung.

**8.** Fraktionierungsvorrichtung nach Anspruch 7, die als Vorbehandlungsvorrichtung dient, die zum Herstellen einer Probe für eine Massenanalyse von Proteinen und/oder Peptiden verwendet wird.

**9.** Fraktionierungsvorrichtung nach Anspruch 8, worin die Proteine und/oder Peptide eine von Blut abgeleitete Flüssigkeit, wie etwa Blut, Serum und Plasma, Urin, Ascites, Saliva, Tränenflüssigkeit, Kammerwasser, Zerebrospinalflüssigkeit, Fruchtwasser, Pleuraflüssigkeit oder eine in Zellextrakt enthaltene Flüssigkeit sind.

**10.** Fraktionierungsvorrichtung, die für Proteine und/oder Peptide verwendet wird, umfassend:

ein Substrat, das eine Oberfläche aufweist, mit der Proteine und/oder Peptide in Kontakt gebracht werden,
worin das Substrat zumindest einen Teil der Substratoberfläche aufweist, auf den ein hydrophiles Polymer aufgepfropft wird, indem die Oberfläche mit einer wässrigen Lösung eines hydrophilen Polymers, das eine Hydroxylgruppe aufweist, in Kontakt gebracht wird und indem die Oberfläche mit radioaktiven Strahlen bestrahlt wird,
und worin zumindest ein Teil des hydrophilen Polymers Polyvinylalkohol oder ein Copolymer von Polyvinylalkohol ist, sodass die Oberfläche menschliches β2-Mikroglobulin in einer Menge von 3 ng/cm$^2$ oder weniger adsorbiert, wenn eine aus menschlichem β2-Mikroglobulin in einer Konzentration von 200 ng/ml und Rinderserumalbumin in einer Konzentration von 10 μg/ml bestehende wässrige Proteinlösung mit der Substratoberfläche in Kontakt gebracht werden.

**11.** Fraktionierungsvorrichtung nach Anspruch 10, worin das hydrophile Polymer Polyvinylalkohol ist.

**12.** Fraktionierungsvorrichtung nach Anspruch 10, worin das Copolymer von Polyvinylalkohol ein Polyvinylalkohol-Polyvinylacetat-Copolymer ist.

**13.** Fraktionierungsvorrichtung nach Anspruch 12, worin das Polyvinylalkohol-Polyvinylacetat-Copolymer einen Verseifungsgrad in einem Bereich von 0,70 oder mehr bis weniger als 1 aufweist.

**14.** Fraktionierungsvorrichtung nach Anspruch 10, worin das Substrat, in dem die Adsorptionsmenge von menschlichem β2-Mikroglobulin 3 ng/cm$^2$ oder weniger beträgt, eine Membran umfasst.

**15.** Fraktionierungsvorrichtung nach Anspruch 14, worin die Membran eine Form von Hohlfasern aufweist.

**16.** Fraktionierungsvorrichtung nach Anspruch 14, worin das Substrat eines der folgenden ist:

ein Mittel zum Bereitstellen einer Lösung, die Proteine und/oder Peptide enthält;
ein Mittel zum Abtrennen von Proteinen und/oder Peptiden von der Lösung; und
ein Mittel zum Konzentrieren von Proteinen und/oder Peptiden in der Lösung.

**17.** Fraktionierungsvorrichtung nach Anspruch 16, die als Vorbehandlungsvorrichtung dient, die zum Herstellen einer Probe für eine Massenanalyse von Proteinen und/oder Peptiden verwendet wird.

**18.** Fraktionierungsvorrichtung nach Anspruch 17, worin die Proteine und/oder Peptide eine von Blut abgeleitete Flüssigkeit, wie etwa Blut, Serum und Plasma, Urin, Ascites, Saliva, Tränenflüssigkeit, Kammerwasser, Zerebrospinalflüssigkeit, Fruchtwasser, Pleuraflüssigkeit oder eine in Zellextrakt enthaltene Flüssigkeit sind.

**19.** Fraktionierungsvorrichtung, die für Proteine und/oder Peptide verwendet wird, umfassend:

ein Mittel zum Bereitstellen einer Lösung, die Proteine und/oder Peptide enthält;
ein Mittel zum Abtrennen von Proteinen und/oder Peptiden von der Lösung; und

ein Mittel zum Konzentrieren von Proteinen und/oder Peptiden in der Lösung,
worin zumindest auf einen Teil der Oberfläche der Fraktionierungsvorrichtung, mit der Proteine und/oder Peptide in Kontakt gebracht werden, ein hydrophiles Polymer aufgepfropft wird, indem die Oberfläche mit einer wässrigen Lösung eines hydrophilen Polymers, das eine Hydroxylgruppe aufweist, in Kontakt gebracht wird und indem die Oberfläche mit radioaktiven Strahlen bestrahlt wird, und das hydrophile Polymer eines von Polyvinylalkohol, Copolymeren davon und Pfropfpolymeren davon ist.

**Revendications**

1. Dispositif de fractionnement utilisé pour des protéines et/ou des peptides, comprenant :

   un substrat ayant une surface au contact de laquelle des protéines et/ou des peptides sont amenés,
   dans lequel le substrat comporte au moins une partie de la surface où un polymère hydrophile est greffé sur celle-ci par mise en contact de la surface avec une solution de polymère hydrophile aqueuse ayant un groupe hydroxyle et par irradiation de la surface avec un rayonnement radioactif,
   et dans lequel au moins une partie du polymère hydrophile est de l'alcool polyvinylique ou un copolymère d'alcool polyvinylique, de sorte que la surface adsorbe de la sérumalbumine bovine en une quantité de 50 $ng/cm^2$ ou moins par rapport à la surface du substrat, quand une solution de sérumalbumine bovine de 1000 $\mu g/ml$ est amenée à son contact.

2. Dispositif de fractionnement selon la revendication 1, dans lequel le polymère hydrophile est de l'alcool polyvinylique.

3. Dispositif de fractionnement selon la revendication 1, dans lequel le copolymère d'alcool polyvinylique est un copolymère d'alcool polyvinylique-acétate polyvinylique.

4. Dispositif de fractionnement selon la revendication 3, dans lequel le copolymère d'alcool polyvinylique-acétate polyvinylique a un degré de saponification allant de 0,70 ou plus à moins de 1.

5. Dispositif de fractionnement selon la revendication 1, dans lequel le substrat dans lequel la quantité d'adsorption de la sérumalbumine bovine est de 50 $ng/cm^2$ ou moins est une membrane.

6. Dispositif de fractionnement selon la revendication 5, dans lequel la membrane a une forme de fibres creuses.

7. Dispositif de fractionnement selon la revendication 6, dans lequel le substrat est l'un des suivantes :

   un moyen permettant d'alimenter une solution contenant des protéines et/ou des peptides ;
   un moyen permettant de séparer des protéines et/ou des peptides de la solution ; et
   un moyen permettant de concentrer des protéines et/ou des peptides dans la solution.

8. Dispositif de fractionnement selon la revendication 7, qui sert de dispositif de prétraitement utilisé pour préparer un échantillon en vue d'une analyse de masse sur des protéines et/ou des peptides.

9. Dispositif de fractionnement selon la revendication 8, dans lequel les protéines et/ou les peptides sont un fluide dérivé de sang, tel que du sang, du sérum et du plasma, de l'urine, de l'ascite, de la salive, du liquide lacrymal, de l'humeur aqueuse, du liquide céphalo-rachidien, du liquide amniotique, du liquide pleural, ou un fluide contenu dans de l'extrait cellulaire.

10. Dispositif de fractionnement utilisé pour des protéines et/ou des peptides, comprenant :

   un substrat ayant une surface au contact de laquelle des protéines et/ou des peptides sont amenés,
   dans lequel le substrat comporte au moins une partie de la surface du substrat où un polymère hydrophile est greffé sur celle-ci par mise en contact de la surface avec une solution de polymère hydrophile aqueuse ayant un groupe hydroxyle et par irradiation de la surface avec un rayonnement radioactif,
   et dans lequel au moins une partie du polymère hydrophile est de l'alcool polyvinylique ou un copolymère d'alcool polyvinylique, de sorte que la surface adsorbe de la $\beta 2$-microglobuline humaine en une quantité de 3 $ng/cm^2$ ou moins quand une solution aqueuse de protéines constituée de $\beta 2$-microglobuline humaine ayant une concentration de 200 ng/ml et de sérumalbumine bovine ayant une concentration de 10 $\mu g/ml$ est amenée

au contact de la surface du substrat.

**11.** Dispositif de fractionnement selon la revendication 10, dans lequel le polymère hydrophile est de l'alcool polyvinylique.

**12.** Dispositif de fractionnement selon la revendication 10, dans lequel le copolymère d'alcool polyvinylique est un copolymère d'alcool polyvinylique-acétate polyvinylique.

**13.** Dispositif de fractionnement selon la revendication 12, dans lequel le copolymère d'alcool polyvinylique-acétate polyvinylique a un degré de saponification allant de 0,70 ou plus à moins de 1.

**14.** Dispositif de fractionnement selon la revendication 10, dans lequel le substrat dans lequel la quantité d'adsorption de la $\beta$2-microglobuline humaine est de 3 $ng/cm^2$ ou moins comprend une membrane.

**15.** Dispositif de fractionnement selon la revendication 14, dans lequel la membrane a une forme de fibres creuses.

**16.** Dispositif de fractionnement selon la revendication 14, dans lequel le substrat est l'un des suivantes :

un moyen permettant d'alimenter une solution contenant des protéines et/ou des peptides ;
un moyen permettant de séparer des protéines et/ou des peptides de la solution ; et
un moyen permettant de concentrer des protéines et/ou des peptides dans la solution.

**17.** Dispositif de fractionnement selon la revendication 16, qui sert de dispositif de prétraitement utilisé pour préparer un échantillon en vue d'une analyse de masse sur des protéines et/ou des peptides.

**18.** Dispositif de fractionnement selon la revendication 17, dans lequel les protéines et/ou les peptides sont un fluide dérivé de sang, tel que du sang, du sérum et du plasma, de l'urine, de l'ascite, de la salive, du liquide lacrymal, de l'humeur aqueuse, du liquide céphalo-rachidien, du liquide amniotique, du liquide pleural, ou un fluide contenu dans de l'extrait cellulaire.

**19.** Dispositif de fractionnement qui est utilisé pour des protéines et/ou des peptides, comprenant :

un moyen permettant d'alimenter une solution contenant des protéines et/ou des peptides ;
un moyen permettant de séparer des protéines et/ou des peptides de la solution ; et
un moyen permettant de concentrer des protéines et/ou des peptides dans la solution,
dans lequel au moins une partie de la surface du dispositif de fractionnement au contact de laquelle des protéines et/ou des peptides sont amenés est greffée avec un polymère hydrophile par mise en contact de la surface avec une solution de polymère hydrophile aqueuse ayant un groupe hydroxyle et par irradiation de la surface avec un rayonnement radioactif et le polymère hydrophile est l'un quelconque parmi de l'alcool polyvinylique, des copolymères de celui-ci et des polymères greffés de celui-ci.

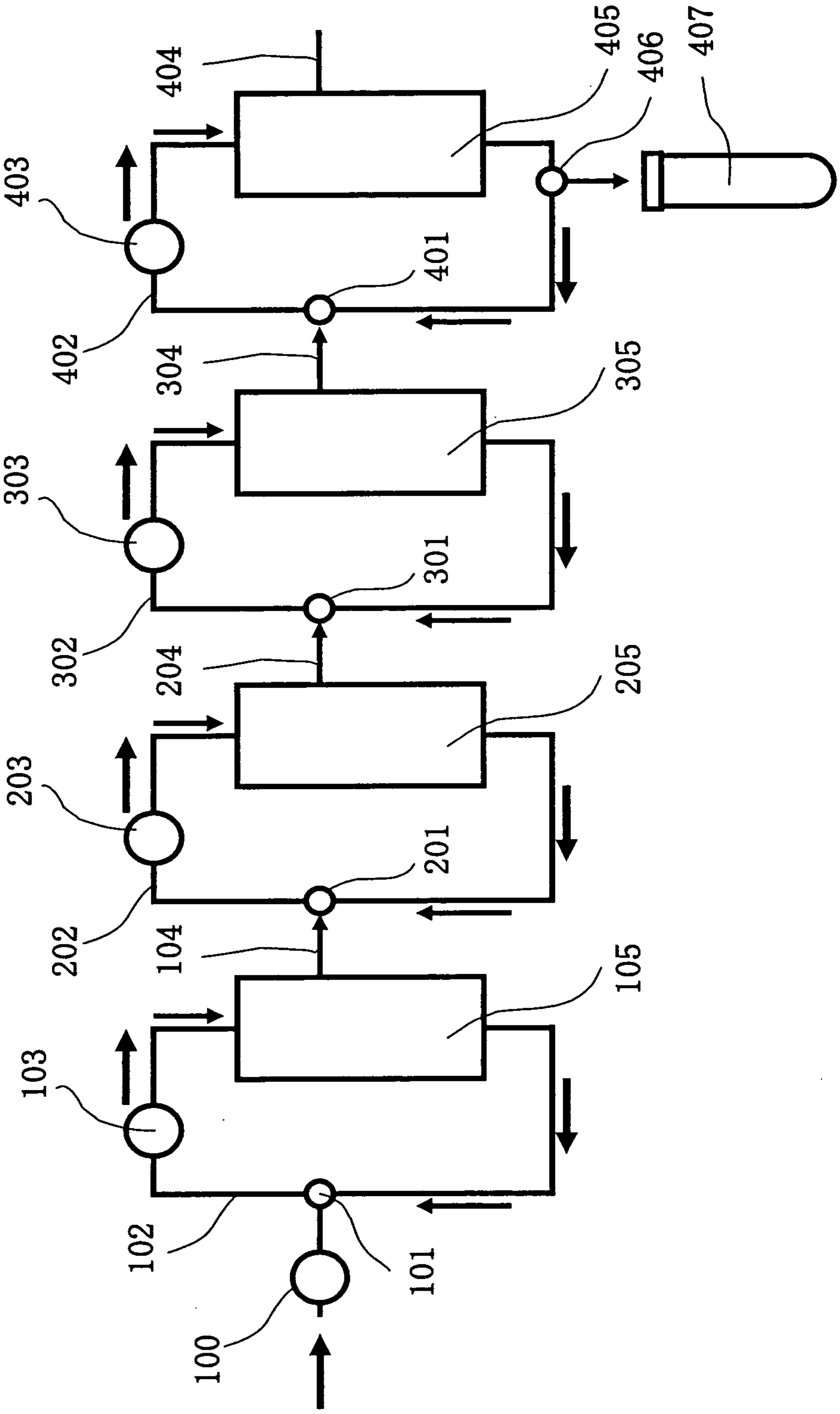
100
101
102
103
104
105
201
202
203
204
205
301
302
303
304
305
401
402
403
404
405
406
407

図1 Fig. 1

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP 2002542163 A
- JP 2003130882 A
- JP 58040323 A
- JP 3297707 B
- WO 2004026931 A
- US 20030068317 A
- WO 0027496 A


### Non-patent literature cited in the description


- The human plasmaproteome: history, character, and diagnostic prospects. **ANDERSON NL ; ANDERSON NG.** Molecular & Cellular Proteomics. The American Society for Biochemistry and Molecular Biology, Inc, 2002, vol. 1, 845-867
- The Japanese Biochemical Society. New Biochemical Experiments. TOKYO KAGAKUDOZIN CO., LTD, 1990, vol. 1